# FASCICULE DE BREVET EUROPEEN

(11) **EP 3 317 017 B1**
(45) Date de publication et mention de la délivrance du brevet: **09.09.2020**
(21) Numéro de dépôt: 16750921.5
(22) Date de dépôt: 01.07.2016
(51) Int. Cl.: B01L 3/00, G01N 33/487, G01N 21/84, A61B 10/00

(54) **APPAREIL D'ANALYSE D'UN ÉCHANTILLON LIQUIDE COMPORTANT UN DISPOSITIF DE BLOCAGE ET DE DÉSENGAGEMENT**
ANALYSEVORRICHTUNG EINER FLÜSSIGEN PROBE, EIN ELEMENT ZUR BLOCKIERUNG UND UNBLOCKIERUNG ENTHALTEND
DEVICE FOR THE ANALYSIS OF A LIQUID SAMPLE COMPRISING A BLOCKING AND UNBLOCKING ELEMENT

(30) Priorité: 03.07.2015 FR 1556309
(43) Date de publication de la demande: 09.05.2018
(73) Titulaire: Avalun, 38000 Grenoble (FR)
(72) Inventeur: POUTEAU, Patrick, 38240 Meylan (FR); POHER, Vincent, 62340 Guines (FR); LAURENS, Paul, 38260 Gillonnay (FR)
(74) Mandataire: GIE Innovation Competence Group
(86) Numéro de dépôt international: PCT/FR2016/051683
(87) Numéro de publication internationale: WO 2017/006037

(56) Documents cités:
- EP-A2- 0 308 770
- US-A- 5 231 576
- US-A- 5 800 781

## Description

### DOMAINE TECHNIQUE

Le domaine de l'invention est celui de l'analyse d'échantillon liquide, par exemple la détection de la présence d'analytes dans un liquide biologique et l'évaluation de leur concentration. L'invention porte sur un appareil d'analyse adapté à coopérer avec un dispositif de collecte d'un échantillon liquide et comportant un dispositif de blocage et de désengagement du dispositif de collecte, ainsi que sur un procédé d'analyse de l'échantillon liquide collecté.

### ÉTAT DE LA TECHNIQUE ANTÉRIEURE

On connaît du document US2007/0202007 un système d'analyse d'un échantillon liquide comportant un dispositif de collecte avec lequel un appareil d'analyse est adapté à coopérer. L'appareil d'analyse comporte un logement situé au niveau d'une paroi latérale permettant l'insertion d'une partie du dispositif de collecte dans le but d'effectuer l'analyse de l'échantillon liquide ainsi qu'un dispositif de détection électrochimique d'un paramètre représentatif de l'échantillon liquide. Le dispositif de collecte se présente sous la forme d'une bande munie de deux électrodes qui s'étendent d'une première extrémité de la bande jusqu'à une zone de mesure dans laquelle se trouvent des réactifs destinés à réagir avec l'échantillon liquide. Le dispositif de collecte comporte, au niveau d'une deuxième extrémité opposée à la première, une surface de réception de l'échantillon liquide qui communique fluidiquement avec la zone de mesure par l'intermédiaire d'un conduit fluidique. Le document US5800781 divulgue un système similaire d'analyse d'un échantillon liquide.

En pratique, on insère la première extrémité de la bande de collecte dans le logement de l'appareil d'analyse, puis on dépose l'échantillon liquide sur la surface de réception de manière à permettre la migration capillaire de celui-ci jusqu'à la zone de mesure, de sorte que l'échantillon liquide réagisse avec les réactifs présents. L'appareil d'analyse applique ensuite une différence de potentiel entre les électrodes de la bande de collecte et un paramètre représentatif de l'échantillon liquide est mesuré à partir des électrodes puis analysé. Cependant, l'appareil d'analyse ne comporte pas de dispositif de blocage et de désengagement permettant de maintenir le dispositif de collecte dans une position de blocage une fois inséré dans le logement, dans le but d'éviter tout déplacement longitudinal du dispositif de collecte dans le logement.

### EXPOSÉ DE L'INVENTION

L'invention a pour objectif de remédier au moins en partie aux inconvénients de l'art antérieur, et plus particulièrement de proposer un appareil d'analyse d'un échantillon liquide adapté à coopérer avec un dispositif de collecte dudit échantillon liquide, comprenant :
- un boitier délimitant un espace intérieur et comprenant une ouverture traversante débouchant sur un logement adapté à recevoir un corps principal du dispositif de collecte et disposé dans l'espace intérieur en s'étendant longitudinalement à partir de l'ouverture suivant un axe longitudinal dit d'insertion du dispositif de collecte,
- un dispositif de détection et d'analyse d'au moins un paramètre représentatif de l'échantillon liquide.

Selon l'invention, l'appareil d'analyse comporte un dispositif de blocage et de désengagement adapté à assurer le maintien dans une position de blocage du dispositif de collecte lorsque le corps principal est inséré à l'intérieur du logement, et comprenant :
- un organe de désengagement adapté à exercer une force de sortie sur le corps principal du dispositif de collecte, dans une direction sensiblement parallèle à l'axe longitudinal du logement et orientée vers l'ouverture du logement ;
- un organe de blocage comportant au moins une portion de butée adaptée à être au contact d'une portion d'appui du corps principal du dispositif de collecte, de manière à immobiliser le dispositif de collecte alors soumis à la force de sortie suivant l'axe longitudinal du logement

Le dispositif de collecte est de préférence amovible, c'est-à-dire qu'il peut être retiré, désengagé voire éjecté de l'appareil d'analyse, et plus précisément du boîtier de l'appareil d'analyse.

Certains aspects préférés mais non limitatifs de cet appareil d'analyse sont les suivants :
L'appareil d'analyse peut comporter une surface d'appui, fixe par rapport au logement, et disposée de manière à délimiter en partie celui-ci en s'étendant de manière parallèle à l'axe longitudinal du logement et de manière à pouvoir être au contact d'une partie du corps principal contenant une chambre fluidique de mesure du dispositif de collecte lorsque celui-ci est inséré dans le logement.

L'organe de blocage peut être mobile vis-à-vis du logement de manière sensiblement transversale à l'axe longitudinal de celui-ci et adapté à exercer une force d'appui sur le corps principal en direction de la surface d'appui lorsque celui-ci est inséré dans le logement.

L'organe de blocage peut comporter une surface de contact orientée vers le logement présentant une partie d'écartement proximale vis-à-vis de l'ouverture du logement et inclinée vis-à-vis de l'axe longitudinal du logement, et une partie d'appui distale, entre lesquelles se situe la portion de butée.

Le dispositif de blocage et de désengagement peut comporter un organe de déblocage dont l'actionnement est adapté à provoquer la mise en mouvement de l'organe de blocage de manière à provoquer un échappement de la butée entre la portion de butée de celui-ci et la portion d'appui du dispositif de collecte, conduisant à un retrait du corps principal vis-à-vis du logement sous l'effet de la force de sortie.

L'appareil d'analyse peut comporter au moins un élément d'écartement disposé longitudinalement le long d'une bordure de la surface d'appui et en saillie vis-à-vis de celle-ci en direction du logement, dont les extrémités longitudinales sont biseautées, de manière à écarter le corps principal vis-à-vis de la surface d'appui lors du déplacement de celui-ci dans le logement selon l'axe longitudinal.

Le dispositif de détection et d'analyse peut comporter un capteur optique situé d'un côté de la surface d'appui opposé au logement, la surface d'appui étant transparente à des faisceaux lumineux provenant de la chambre de mesure lorsque le corps principal du dispositif de collecte est inséré dans le logement.

Le dispositif de détection et d'analyse peut comporter un capteur optique situé à une distance longitudinale vis-à-vis de l'ouverture du logement inférieure ou égale à 2cm et/ou à une distance transversale vis-à-vis du logement inférieure ou égale à 2cm.

Un dispositif de chauffage peut comprendre un élément chauffant adapté à transmettre de la chaleur au corps principal par l'intermédiaire de la surface d'appui lorsque ce dernier est inséré dans le logement et au contact de la surface d'appui.

Le dispositif de détection et d'analyse peut comporter au moins une source de lumière et un capteur optique disposés de part et d'autre du logement suivant un axe d'éclairement du corps principal du dispositif de collecte lorsqu'il est inséré dans le logement, l'organe de blocage comportant une partie creuse positionnée entre la source de lumière et le capteur optique permettant la propagation des faisceaux d'illumination suivant l'axe d'éclairement au travers de ladite partie creuse.

Le boitier peut comporter une paroi supérieure et une paroi inférieure reliées l'une à l'autre par une paroi latérale présentant une largeur entre lesdites parois inférieure aux dimensions des parois supérieure et inférieure, l'ouverture du logement étant située au niveau de la paroi supérieure du boitier et le logement s'étendant de manière sensiblement orthogonale à celle-ci en direction de la paroi inférieure.

La paroi supérieure du boitier peut comprendre un écran d'affichage.

L'invention porte également sur un système d'analyse d'un échantillon liquide, comportant un appareil d'analyse selon l'une quelconque des caractéristiques précédentes, caractérisé en ce qu'il comporte en outre un dispositif de collecte dudit l'échantillon liquide, comprenant :
- un corps principal s'étendant suivant un axe longitudinal et comportant une chambre fluidique de mesure, apte à être inséré longitudinalement dans le logement suivant l'axe longitudinal d'insertion de celui-ci ;
- une surface de réception, destinée à être située hors du logement pour recevoir l'échantillon liquide lorsque le corps principal est inséré dans le logement, assemblée au corps principal et comprenant un orifice de collecte communiquant fluidiquement avec la chambre de mesure.

La surface de réception peut s'étendre de manière sensiblement orthogonale à l'axe longitudinal du corps principal.

Le logement de l'appareil d'analyse et le dispositif de collecte peuvent être dimensionnés l'un et l'autre de sorte que, lorsque le corps principal du dispositif de collecte est inséré dans le logement, la distance entre l'orifice de collecte et la chambre de mesure est approximativement égale à la distance entre l'ouverture du logement et la chambre de mesure, et est de préférence inférieure ou égale à 2cm.

Le dispositif de collecte peut comporter une partie de collecte assemblée au corps principal et dont une surface forme la surface de réception, cette partie de collecte étant au contact d'une paroi du boitier en bordure de l'ouverture du logement lorsque le corps principal du dispositif de collecte est inséré dans le logement.

La portion d'appui du corps principal du dispositif de collecte est formée par un épaulement situé entre une première partie dite centrale contenant une chambre de mesure et une extrémité distale du corps principal vis-à-vis de la chambre de mesure.

### BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :
la figure 1a est une vue en perspective d'un système d'analyse comprenant un appareil d'analyse d'un échantillon liquide et un dispositif de collecte de l'échantillon liquide inséré dans ce dernier, et la figure 1b est une vue schématique en coupe d'une partie du système d'analyse représenté sur la figure 1a où le dispositif de collecte est inséré dans l'appareil d'analyse ;
la figure 2a est une vue de face d'un exemple de dispositif de collecte représenté sur la figure 1a, et la figure 2b est une vue en perspective illustrant les deux parties mâle et femelle formant le dispositif de collecte représenté sur la figure 2a ;
les figures 3a à 3c sont des vues schématiques du dispositif de blocage et de désengagement de l'appareil d'analyse, suivant différents angles de vue ;
les figures 4a et 4b sont des vues schématiques du dispositif de collecte et du dispositif de blocage et de désengagement de l'appareil d'analyse, lorsque le dispositif de collecte est en cours d'insertion (figure 4a) et lorsqu'il est en position de blocage (figure 4b) ;
les figures 5a et 5b sont des vues en perspective d'un exemple du dispositif de blocage et de désengagement ;
la figure 6 est une vue schématique en coupe d'un exemple de capteur optique muni d'un dispositif de chauffage.

### EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures.

L'invention porte sur un appareil d'analyse d'un échantillon liquide ainsi que sur un système d'analyse comportant un tel appareil. L'échantillon liquide peut être issu d'un liquide biologique, tel que du sang, de la salive, de l'urine, du liquide interstitiel, voire de tout autre type de liquide dont on cherche à détecter et analyser un paramètre représentatif. L'analyse de l'échantillon liquide revient à évaluer un ou plusieurs paramètres représentatifs de l'échantillon liquide. Ces paramètres peuvent être, ou être issus, des propriétés optiques (par exemple le taux d'absorption, la colorimétrie, des figures d'interférence...), électriques (par exemple l'impédance, la conductance...), chimiques (par exemple la présence et la concentration en analytes...) de l'échantillon liquide. Il peut ainsi s'agir de détecter et d'évaluer la présence et la concentration d'un ou de plusieurs analytes présents dans l'échantillon liquide, par exemple des particules biologiques telles que des cellules, des bactéries, des protéines ou l'un de leurs constituants, ou autres. L'échantillon liquide peut comporter un ou plusieurs réactifs susceptibles de modifier de manière proportionnelle ou non une ou plusieurs desdites propriétés de l'échantillon liquide à analyser.

La figure la illustre un système d'analyse 1 selon un mode de réalisation, comprenant un appareil d'analyse 100 d'échantillon liquide dans lequel est inséré un dispositif de collecte 200 dudit échantillon liquide.

On définit ici et pour la suite de la description un repère orthonormé tridimensionnel, où l'axe X est orienté suivant la largeur de l'appareil d'analyse, l'axe Y suivant sa longueur et l'axe Z suivant son épaisseur.

L'appareil d'analyse 100 comporte un boitier 110 formé de parois supérieure 111 et inférieure 112 reliées l'une à l'autre par une paroi latérale 113, qui délimite un espace intérieur. La paroi supérieure 111 est ici sensiblement coplanaire au plan (X,Y) et peut comporter un écran d'affichage 114 ainsi que des touches 115 de sélection de différents modes d'utilisation de l'appareil. La paroi inférieure 112 est ici sensiblement coplanaire au plan (X,Y). La paroi latérale 113 s'étend sur tout le contour de l'appareil et relie les parois supérieure 111 et inférieure 112 l'une par l'autre. L'appareil d'analyse 100 présente ici une épaisseur inférieure à sa largeur et à sa longueur.

Le dispositif de collecte 200 est inséré dans un logement de l'appareil d'analyse 100, ici au niveau de la paroi supérieure 111, de sorte que n'apparait hors de l'appareil qu'une partie dite de collecte 210 du dispositif dont une surface supérieure 211 est destinée à recevoir l'échantillon liquide. Dans cet exemple, cette surface dite de réception 211 est avantageusement sensiblement orthogonale à l'axe longitudinal de la partie du dispositif de collecte insérée dans le logement ainsi qu'à celui du logement, mais elle pourrait être sensiblement parallèle à l'axe de la partie du dispositif de collecte insérée notamment lorsque l'ouverture se situe au niveau de la paroi latérale 113 du boitier. Elle comprend un orifice 212 destiné à permettre l'introduction de l'échantillon liquide à l'intérieur du dispositif de collecte 200 et ainsi à l'intérieur de l'appareil d'analyse 100, dans le but de procéder à l'analyse de l'échantillon.

Par dispositif de collecte inséré dans l'appareil d'analyse, ou plus précisément dans un logement prévu à cet effet, on entend qu'une partie du dispositif est engagée ou insérée dans le logement et qu'une autre partie du dispositif, telle que la partie de collecte, est située hors du logement. De plus, par surface de réception sensiblement orthogonale, respectivement parallèle, à l'axe longitudinal du logement, ou axe d'insertion, on entend que la surface de réception présente, lorsque le dispositif de collecte est inséré dans l'appareil d'analyse, un plan tangent à la surface, au niveau de l'orifice de collecte, sensiblement orthogonal, respectivement parallèle, à l'axe d'insertion.

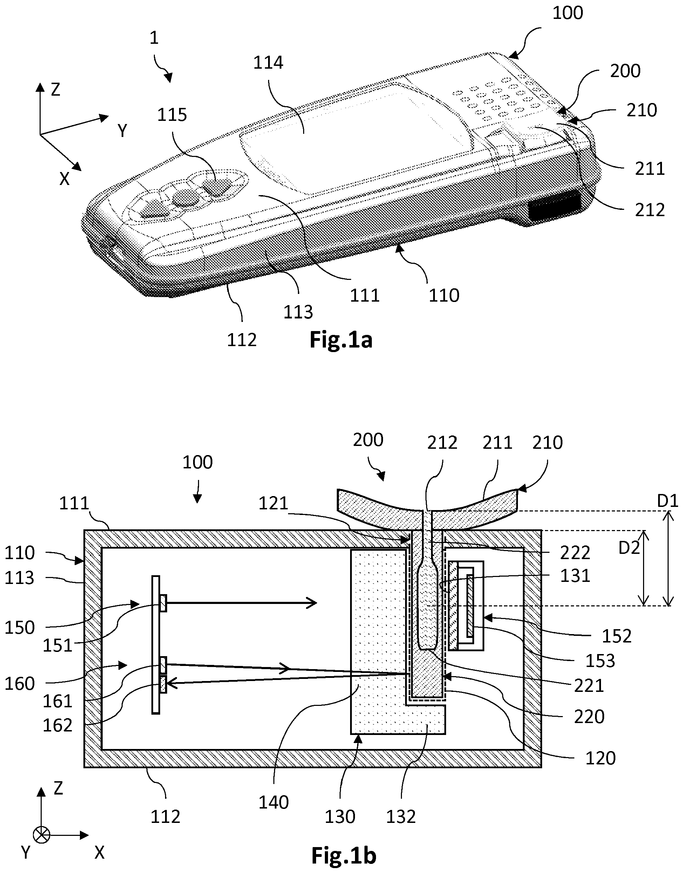

La figure 1b est une vue schématique en coupe selon le plan (X,Z) d'une partie du système d'analyse représenté sur la figure la dans laquelle le dispositif de collecte est inséré dans l'appareil d'analyse.

Le boitier 110 de l'appareil d'analyse délimite un espace intérieur dans lequel sont présents un logement 120 destiné à recevoir une partie du dispositif de collecte 200 en vue de procéder à l'analyse de l'échantillon liquide, un dispositif de blocage et de désengagement 130 du dispositif de collecte et un dispositif de détection et d'analyse 150 d'une ou plusieurs propriétés caractéristiques de l'échantillon.

Le logement 120 est adapté à recevoir une partie du dispositif de collecte dans une position permettant la détection et l'analyse de l'échantillon liquide. Il communique avec l'environnement par une ouverture 121 traversante pratiquée ici au niveau de la paroi supérieure 111 du boitier et s'étend suivant un axe longitudinal dit d'insertion à partir de l'ouverture 121 en direction de la paroi inférieure 112. Le logement 120 est en partie délimité par un dispositif de blocage et de désengagement 130, qui comporte ici une surface d'appui 131 qui s'étend longitudinalement le long d'un côté du logement, un organe de blocage 140 disposé du côté du logement opposé à la surface d'appui 131, et un organe de désengagement 132 situé en regard de la partie inférieure du logement 120, c'est-à-dire la partie distale du logement 120 vis-à-vis de l'ouverture 121.

L'exemple de dispositif de collecte 200 ici représenté est formé d'une partie de collecte 210 dont une surface supérieure forme la surface de réception 211 de l'échantillon liquide, et un corps principal 220 qui s'étend longitudinalement de manière sensiblement orthogonale à la partie de collecte 210. Il est bien précisé qu'un dispositif de collecte ayant une surface de réception 211 s'étendant de manière parallèle au corps principal pourrait également être utilisé. Dans cet exemple, l'axe longitudinal du corps principal 220 est sensiblement orthogonal à un plan tangent à la surface de réception 211 au niveau de l'orifice de collecte 212. Le corps principal 220 comporte une chambre de mesure 221 qui communique fluidiquement avec l'orifice de collecte 212 par un conduit fluidique 222. Sur la figure, le dispositif de collecte 200 est inséré dans le logement 120 de sorte que le corps principal 220 est engagé dans le logement et la partie de collecte 210 est située à l'extérieur du logement. Il est avantageux que celle-ci repose ou soit en contact avec la paroi du boitier 110, pour assurer d'une part un positionnement précis du dispositif de collecte 200 dans l'appareil d'analyse 100 suivant l'axe d'insertion, et d'autre part un appui de la partie de collecte lors de la phase de dépôt de l'échantillon liquide et offrir ainsi une meilleure répartition des contraintes mécaniques.

L'appareil d'analyse 100 comporte un dispositif de détection et d'analyse 150 d'une ou plusieurs propriétés caractéristiques de l'échantillon, qui peut être de type électrique, chimique et/ou optique. Dans cet exemple, le dispositif de détection 150 est de type optique et comporte une ou plusieurs sources de lumière 151 et un capteur optique 152. Une source de lumière 151 est ici disposée de manière à pouvoir illuminer la chambre de mesure 221 insérée dans le logement 120, suivant une direction d'éclairement, ici parallèle à l'axe X. Le capteur optique 152 comporte un photodétecteur 153 disposé derrière la surface d'appui 131 suivant la direction d'éclairement, celle-ci étant alors réalisée en un matériau transparent aux faisceaux incidents provenant de la chambre de mesure 221 éclairée. Comme il sera détaillé plus loin, il est avantageux que le dispositif de détection 150 réalise une détection optique par la technique dite d'imagerie sans lentille. Par ailleurs, on note ici que l'organe de blocage 140, placé entre la source de lumière 151 et le capteur optique 152, est structuré de manière à permettre le passage des faisceaux d'illumination en direction du logement 120, sans induire de perturbation optique desdits faisceaux.

Le dispositif de détection et d'analyse 150 comporte en outre une unité d'analyse (non représentée) qui assure le traitement des données mesurées. L'unité d'analyse est reliée à l'écran d'affichage 114 pour permettre l'affichage d'informations à destination de l'utilisateur. De manière classique, l'unité d'analyse comporte un processeur et une mémoire apte à stocker un logiciel de traitement des données mesurées. Cette unité d'analyse peut également assurer le stockage de données, la communication avec des éléments extérieurs à l'appareil d'analyse (système informatique...), la gestion des interfaces boutons, l'alimentation électrique du dispositif de détection et d'analyse 150, etc. Une batterie d'alimentation électrique peut être prévue à l'intérieur du boitier 110 de l'appareil 100.

Comme illustré sur la figure, un dispositif de lecture optique 160 peut également être prévu, par exemple pour assurer la lecture d'informations présentes sur le corps principal 220 du dispositif de collecte 200 engagé dans le logement 120. Ces informations peuvent être contenues dans un code QR (pour *Quick Response,* en anglais), ou code Datamatrix, et concerner par exemple le type d'échantillon liquide à analyser, le type de détection à effectuer, le type de réactifs présents dans la chambre de mesure, la date de péremption du dispositif de collecte, le numéro du lot de fabrication, etc.... Ce dispositif de lecture optique 160 comprend au moins une source de lumière 161 et un capteur optique 162, de préférence un photodétecteur matriciel. L'exemple représenté montre un dispositif destiné à fonctionner en réflexion, le capteur optique 162 étant placé du même côté que la source de lumière 161 vis-à-vis du logement 120, mais un agencement pour un fonctionnement en transmission est possible.

Les figures 1a et 1b montrent que l'appareil d'analyse 100 et le dispositif de collecte 200 sont adaptés l'un et l'autre pour permettre l'insertion d'une partie du dispositif de collecte 200 dans le logement 120 suivant un axe d'insertion, ou axe longitudinal, sensiblement orthogonal à la surface de réception 211.

Dans cet exemple, la surface de réception est sensiblement orthogonale à l'axe longitudinal du corps principal et à l'axe longitudinal du logement. Elle est donc sensiblement coplanaire à la bordure de l'ouverture du logement, et peut donc être située, lorsque le dispositif de collecte est inséré dans le logement, au plus près du boitier. Cela permet de minimiser fortement la partie du dispositif de collecte situé hors de l'appareil d'analyse et donc notamment de réduire la longueur nécessaire du conduit fluidique qui relie l'orifice de collecte à la chambre de mesure, et ainsi de diminuer le volume nécessaire d'échantillon liquide. Par ailleurs, en réduisant la partie du dispositif de collecte située hors de l'appareil d'analyse, on limite les risques de fausse manipulation du dispositif de collecte lors de l'étape de détection, ces fausses manipulations étant susceptibles de perturber la détection et ainsi de fausser les résultats de l'analyse.

Il est avantageux que l'ouverture du logement soit située au niveau de la paroi supérieure du boitier et que le logement s'étende de manière sensiblement orthogonale à celle-ci en direction de la paroi inférieure. Les parois supérieures et inférieures présentent des dimensions suivant X et Y, ou longueur et largeur, supérieures à la dimension suivant Z, ou épaisseur, de la paroi latérale.

De plus, l'échantillon liquide peut être déposé sur la surface de réception en exerçant une force d'appui sur celle-ci, par exemple en appuyant le doigt d'un patient sur cette surface. Le patient exerce donc une force d'appui sur la surface de réception qui sera parallèle à l'axe longitudinal du corps principal. On évite ainsi les efforts de torsion que peuvent subir les bandes de collecte telles que celles décrites précédemment en référence à l'art antérieur. De plus, dans le cas où la partie de collecte est au contact avec la paroi du boitier, la force d'appui est parallèle à l'axe longitudinal du logement, ce qui permet une meilleure répartition des contraintes mécaniques subies par le dispositif de collecte et transmises au boitier. Lors de l'étape de dépôt de l'échantillon liquide sur la surface de réception, celle-ci est avantageusement orientée de manière approximativement horizontale vis-à-vis de l'axe de la gravité, c'est-à-dire sensiblement orthogonale à celui-ci.

On note que la partie de collecte 210 s'étend transversalement en saillie par rapport au corps principal et présente une étendue surfacique de la surface de réception 211 supérieure à l'étendue surfacique d'une section transversale du corps principal 220, notamment au niveau de la chambre de mesure. En effet, le corps principal est transversalement relativement étroit pour être inséré dans un logement de petite dimension alors que la surface de réception est plus importante pour recevoir l'échantillon liquide. A titre d'exemple, l'étendue surfacique d'une section transversale du corps principal au niveau de la chambre de mesure peut être de l'ordre de la dizaine à quelques dizaines de millimètres carré alors que l'étendue surfacique de la surface de réception peut être de l'ordre de la centaine à quelques centaines de millimètres carré. L'étendue de la surface de réception 211 assure une protection de la surface de l'appareil d'analyse 100 et en particulier de l'ouverture 121 du logement 120 en cas de surplus de volume d'échantillon biologique ou d'imprécision au moment du dépôt de l'échantillon.

De plus, le logement de l'appareil d'analyse 100 et le dispositif de collecte 200 peuvent être dimensionnés l'un et l'autre de sorte que, lorsque le corps principal 220 du dispositif de collecte est inséré dans le logement 120, la distance D1 entre l'orifice de collecte 212 et la chambre de mesure 221 est approximativement égale à la distance D2 entre l'ouverture du logement 121 et la chambre de mesure, ces distances étant par exemple inférieures ou égales à 2cm, voire inférieures ou égales à 1cm. La distance D1 peut être mesurée entre l'orifice 212 et le centre longitudinal suivant l'axe Z de la chambre de mesure. De manière similaire, la distance D2 peut être mesurée entre l'ouverture 121, par exemple à la surface débouchante de la paroi du boitier, et le centre longitudinal suivant l'axe Z de la chambre de mesure. Par distances D1 et D2 approximativement égales l'une à l'autre, on entend que l'écart entre ces deux distances est inférieur ou égal à 25% de la distance la plus grande, et de préférence inférieur à 10%. Concrètement, ces distances D1 et D2 sont égales moyennant l'épaisseur de la partie de collecte 210 lorsque celle-ci est au contact avec la bordure de l'ouverture 121. A titre d'exemple, pour une distance D1 égale à 1cm, la distance D2 est égale à 0,8cm pour une épaisseur de partie de collecte de l'ordre de 2mm. On réduit ainsi fortement la longueur du conduit fluidique reliant l'orifice de collecte 212 à l'entrée de la chambre de mesure 221.

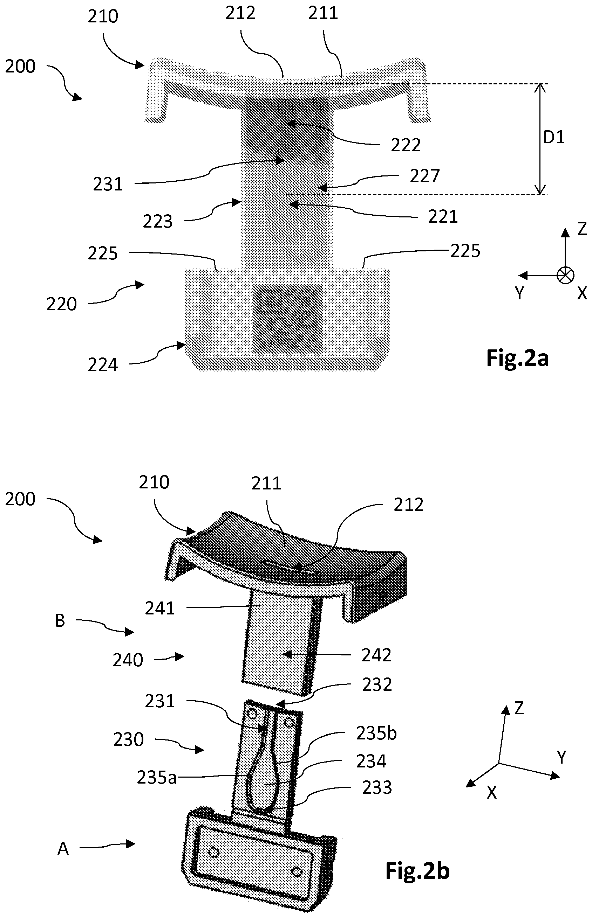

La figure 2a est une vue de face d'un exemple du dispositif de collecte selon un mode de réalisation décrit dans la demande de brevet FR1551725 déposée le 02/03/2015.

Comme mentionné plus haut, le dispositif de collecte 200 comporte une partie de collecte 210 et un corps principal 220. La partie de collecte 210 présente une surface supérieure dite de réception 211 destinée à recevoir l'échantillon liquide. Elle comporte un orifice de collecte 212 permettant l'introduction de l'échantillon liquide déposé à l'intérieur du dispositif de collecte. Cet orifice 212 communique fluidiquement avec une chambre de mesure 221 disposée dans le corps principal 220 par l'intermédiaire d'un conduit fluidique 222.

Le corps principal 220 présente des dimensions adaptées à celles du logement 120. Il comporte une partie centrale 223 assemblée à la partie de collecte 210 en une extrémité, dans laquelle s'étendent le conduit fluidique 222 et la chambre de mesure 221. Cette dernière présente un élargissement de sa largeur, suivant l'axe Y, vis-à-vis du conduit fluidique de manière à augmenter la surface destinée à être illuminée dans le cas d'une détection optique. La partie centrale 223 du corps principal est assemblée en son extrémité opposée à une seconde partie 224 formant un talon de préhension permettant à un utilisateur de manipuler aisément le dispositif. Le talon 224 présente une largeur suivant l'axe Y supérieure à celle de la partie centrale du corps principal, de manière à ce que la paroi latérale suivant l'axe Y comporte au moins une, ici deux portions d'appui 225, sous forme d'épaulement, destinées à coopérer avec le dispositif de blocage de l'appareil d'analyse. Par épaulement, on entend une variation brusque de la section transversale du corps principal afin de former une surface d'appui orientée en direction de la partie de collecte. En variante, le dispositif de collecte peut comporter une portion d'appui, réalisée non pas sous forme d'épaulement mais sous forme d'un évidement pratiqué dans la partie centrale ou le talon du corps principal.

Enfin, le talon comporte ici une zone d'informations inscrites sous forme par exemple d'un code QR ou code datamatrix, par exemple située sous la chambre de mesure.

De plus, il peut comporter au moins une portion en saillie, ici deux portions qui s'étendent transversalement au plan (Y,Z) du talon. Ces portions assurent une fonction de détrompeur permettant d'éviter toute erreur dans l'utilisation ou la manipulation du dispositif de collecte. Le logement présente une forme adaptée permettant l'insertion du corps principal qui comporte de tels détrompeurs.

La figure 2b illustre une vue en perspective de l'exemple de dispositif de collecte représenté sur la figure 2a, réalisé en deux parties, comme décrit dans la demande de brevet FR1551725 déposée le 02/03/2015.

Le dispositif de collecte 200 comporte deux éléments A et B, distincts l'un de l'autre, et destinés à coopérer l'un avec l'autre pour rendre le dispositif opérationnel.

Le premier élément A comprend une partie mâle 230 au niveau de laquelle se situe un canal 231 à section transversale ouverte. Le canal 231 s'étend longitudinalement entre une première extrémité 232, dite d'entrée, apte à recevoir un échantillon liquide, et une seconde extrémité 233. Le canal 231 est formé par une paroi longitudinale 234, appelée paroi de fond, bordée de deux parois latérales 235a, 235b qui forment les flancs du canal 231.

Le second élément B comporte une partie femelle 240 formée d'une paroi périphérique 241 qui délimite transversalement une cavité destinée à loger, ou recevoir, la partie mâle 230. De plus, une partie de la paroi périphérique 241 est destinée, lorsque la partie femelle loge la partie mâle, à former un capot 242 fermant la section transversale du canal 231. La partie femelle 240 est assemblée à la partie de collecte 210 qui s'étend transversalement à l'axe longitudinal de la partie mâle 230 et de la partie femelle 240.

Ainsi, pour rendre opérationnel le dispositif de collecte 200, la partie mâle 230 du premier élément A est introduite dans la partie femelle 240 du second élément B, de sorte que la paroi périphérique 241 ferme la section transversale du canal 231, de préférence sur toute la longueur du canal. Un échantillon liquide peut alors être amené au contact de l'extrémité d'entrée 232 du canal 231 par l'orifice de collecte 212 de la surface de réception 211, ce qui provoque son insertion dans le canal 231 et l'écoulement capillaire de l'échantillon le long du canal 231 en direction de la deuxième extrémité 233.

Il résulte de cette réalisation en deux éléments distincts A et B du dispositif de collecte 200 dont le canal 231 présente une section transversale ouverte au niveau longitudinal : avant insertion de la partie mâle dans la partie femelle, un accès direct à l'intérieur du canal ; après insertion de la partie mâle dans la partie femelle, un meilleur confinement du canal.

L'accès direct à l'intérieur du canal, c'est-à-dire à son volume interne et à au moins une partie de ses surfaces internes, permet de fonctionnaliser et/ou traiter l'intérieur du canal, en partie ou totalement, avec une simplicité et une efficacité que l'art antérieur ne permet pas d'atteindre. Ainsi, dans le cas où on souhaite déposer dans le canal un réactif séché ou lyophilisé destiné à interagir avec l'échantillon liquide, l'accès direct à l'intérieur du canal permet de procéder de manière simple et rapide à un dépôt particulièrement homogène du réactif. En effet, l'intérieur du canal est accessible directement sur toute sa longueur. La précision de positionnement des outils de dépôt du ou des réactifs à sécher ou lyophiliser peut alors être simplifiée. Il est également possible de réaliser simplement un dépôt localisé du ou des réactifs à sécher ou lyophiliser, directement dans une zone désirée du canal (que ce soit à l'entrée, au milieu ou à la sortie du canal). Il est également possible de déposer dans le canal, de manière simple et précise, des électrodes ou une membrane absorbante, voire de fonctionnaliser par liaison chimique l'intérieur du canal, par exemple par immobilisation locale d'une ou plusieurs molécules chimiques ou entités biologiques (protéine, séquence ADN, anticorps, etc...) directement sur une paroi du canal, par exemple par liaison chimique covalente.

Le dispositif de collecte offre également, lorsque la partie femelle loge la partie mâle, un confinement particulièrement efficace du canal, ce qui permet de limiter voire éviter la contamination de l'environnement extérieur par le liquide présent dans le canal ainsi que la pollution du canal à partir de l'extérieur.

Dans cet exemple, la partie mâle 230 est formée d'une plaque présentant une section transversale sensiblement rectangulaire. Le canal 231 est disposé au niveau d'une face longitudinale, dite supérieure, de la plaque, de sorte que l'extrémité d'entrée 232 du canal 231 débouche sur une paroi transversale d'extrémité de la plaque. La plaque présente ici une section transversale rectangulaire, mais tout type de section peut également convenir, par exemple carrée, voire également circulaire. La paroi longitudinale de fond 234 du canal 231, présente avantageusement une dimension, en coupe transversale, supérieure à celle de chacun des flancs 235a, 235b. En d'autres termes, le facteur de forme transversal du canal 231, à savoir le rapport entre la largeur du canal sur sa profondeur, est, dans cet exemple, strictement supérieur à 1, de préférence supérieur à 5, voire à 10.

Dans cet exemple, la paroi périphérique 241 de la partie femelle 240 s'étend longitudinalement, suivant l'axe Z, de manière à former une cavité apte à recevoir et loger, de préférence entièrement, la partie mâle 230. De plus, les dimensions internes de la paroi périphérique 241 sont ajustées de sorte que la surface du pourtour de la partie mâle 230 vienne au contact de la surface interne de la paroi périphérique 241, lorsque la partie femelle 240 loge la partie mâle 230. La paroi périphérique 241 forme ici une cavité de section transversale, dans le plan (X,Y), sensiblement rectangulaire, correspondant à la section transversale rectangulaire de la partie mâle 230. Cependant, toute autre forme, complémentaire de celle de la partie mâle, peut convenir. La cavité s'étend entre une ouverture d'insertion, par laquelle la partie mâle est destinée à être introduite, et l'orifice opposé 212 de collecte apte à recevoir l'échantillon liquide pour l'insertion de celui-ci dans le canal.

Le second élément B comporte en outre la surface de réception 211, ici sous forme d'une coupelle, destinée à recevoir l'échantillon liquide. La partie femelle 240 est assemblée à la surface de réception 211, de sorte que l'orifice de collecte 212 de la cavité débouche au niveau de la surface de réception 211. La surface de réception 211 s'étend en saillie par rapport à la section transversale du corps principal et de manière sensiblement orthogonale à la partie femelle 240, et peut présenter une forme courbe, évasée, plus précisément convexe, pour permettre à faciliter la mise en contact de l'échantillon liquide avec l'orifice de collecte 212. Cette courbe est aussi particulièrement adaptée à la dépose d'une goutte pendante au bout d'un doigt d'un patient, tel qu'elle peut se présenter lors d'une collecte de sang capillaire au bout d'un doigt.

Ainsi, lorsqu'un échantillon liquide est déposé sur la surface de réception 211 et vient au contact de l'orifice de collecte 212, il s'introduit par capillarité dans le canal 231 par son extrémité d'entrée 232 dans une première partie qui forme le conduit fluidique 222 jusqu'à la chambre de mesure 221.

Pour favoriser l'introduction de l'échantillon liquide par capillarité dans le canal 231, les parois du dispositif de collecte présentent idéalement un angle de mouillage inférieur à 90° et idéalement inférieur à 50°. Ceci peut être obtenu en choisissant des matériaux présentant naturellement cet angle de mouillage ou bien par traitement chimique après fabrication des éléments A et B du dispositif. Un traitement au plasma oxygène ou bien un traitement par insolation avec une lampe UV intense en présence d'oxygène ou d'ozone peut être aussi utilisé.

En référence à la figure 2a, au niveau de l'extrémité de la chambre de mesure 221 opposée au conduit fluidique 222, un évent 227 de mise à l'air libre communique avec la chambre de mesure 221 par l'intermédiaire d'un conduit dont la largeur peut être inférieure à celle de la chambre de mesure 221. Ce conduit peut assurer également la fonction d'arrêt d'écoulement du liquide, par sa différence de largeur avec celle de la chambre de mesure, et assure ainsi une fonction de vanne passive.

D'une manière générale, le dispositif de collecte 200 peut être utilisé comme un consommable jetable, autorisant une analyse de l'échantillon par des moyens appropriés, réalisée éventuellement en-dehors d'un laboratoire d'analyse.

Les moyens d'analyse peuvent être basés sur une analyse du signal optique émis par l'échantillon liquide ou modifié par celui-ci, les parois du corps principal 220 autorisant la transmission du signal optique à mesurer, et éventuellement la transmission de faisceaux d'illumination émis par une source de lumière en direction de l'échantillon liquide. Les moyens d'analyse peuvent également être basés sur une analyse électrique de l'échantillon liquide, et peuvent comporter une ou plusieurs électrodes disposées dans la chambre de mesure et reliées électriquement à une source de tension ou de courant lorsque le dispositif de collecte est inséré dans l'appareil 100. Un mode de réalisation des électrodes par dépôt en couche mince des électrodes ainsi que des pistes de contact permettant d'amener la reprise de contact sur le talon 224 du corps principal 220 peut être utilisé.

Le dispositif de collecte, en particulier les parties mâle 230 et femelle 240, peut être réalisé, par exemple, par une technique de moulage ou d'injection d'un matériau plastique tel que du polycarbonate, polypropylène, polyéthylène, cyclo-oléfine-copolymère (COC), cyclo-oléfine-polymère (COP), ou tout autre matériau pouvant convenir. Le matériau formant les parties mâle et femelle est de préférence transparent au rayonnement visible et/ou infrarouge, en particulier lorsqu'une analyse de l'échantillon liquide par des moyens optiques est prévue.

Le dispositif de collecte peut présenter une longueur totale, suivant l'axe Z, de l'ordre de quelques centimètres, par exemple deux centimètres, la coupelle peut présenter une surface de quelques centimètres carrés, par exemple 2cm x 1cm, et l'orifice de collecte une longueur de quelques millimètres, par exemple 5mm, sur une largeur de 0,1mm à 1mm. La partie mâle peut présenter une longueur et une largeur de quelques millimètres, par exemple 10mm x 5mm, pour une épaisseur de quelques centaines de microns voire quelques millimètres, par exemple 1mm. Le canal peut présenter une longueur de quelques millimètres ou centimètres, une largeur de quelques centaines de microns à quelques millimètres, et une épaisseur de l'ordre de quelques dizaines de microns à quelques millimètres. A titre d'exemple, la chambre de mesure peut présenter une longueur (suivant l'axe Z) de 6,5mm, une largeur (suivant l'axe Y) de 3mm dans sa zone la plus large, et une profondeur (suivant l'axe X) de 150µm. Ces ordres de grandeur ne sont donnés qu'à titre illustratif.

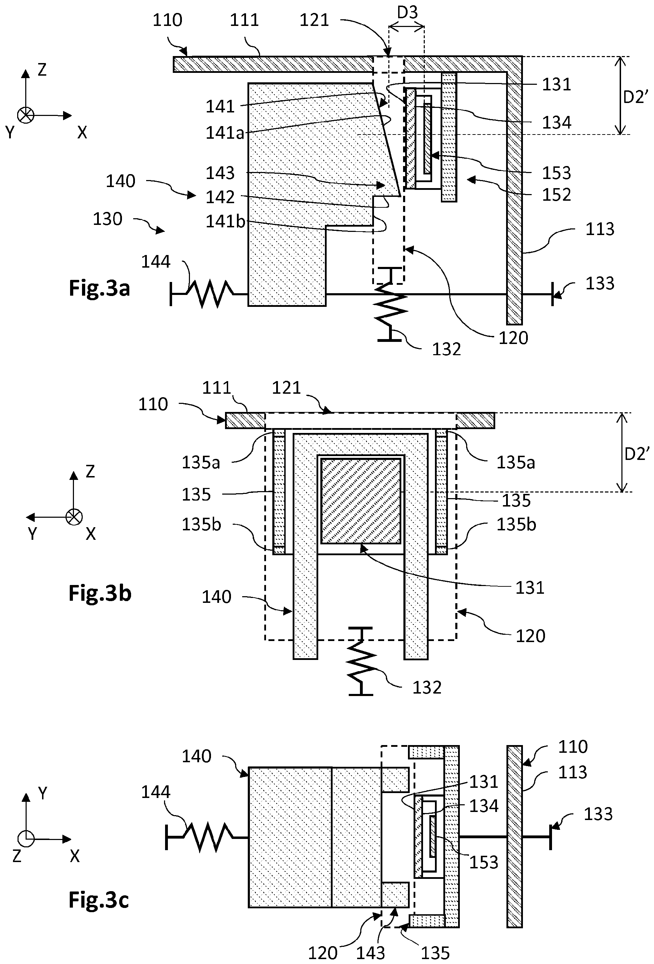

Les figures 3a à 3c sont des vues schématiques d'une partie de l'appareil d'analyse adapté à recevoir un dispositif de collecte et comportant un dispositif de blocage et de désengagement de ce dispositif de collecte, par exemple celui décrit en référence aux figures 2a et 2b ou un dispositif de collecte où la partie de collecte serait parallèle et coplanaire au corps principal.

Le dispositif de blocage et de désengagement 130 est adapté à assurer le maintien dans une position de blocage du dispositif de collecte lorsque le corps principal 220 est inséré à l'intérieur du logement 120. Pour cela, il comprend :
- un organe de désengagement 132 adapté à exercer une force de sortie sur le corps principal 220 du dispositif de collecte, dans une direction sensiblement parallèle à l'axe longitudinal du logement et orientée vers l'ouverture 121 du logement ; et
- un organe de blocage 140 comportant au moins une portion de butée 143 adaptée à être au contact d'une portion d'appui 225 du corps principal 220 du dispositif de collecte, de manière à immobiliser le dispositif de collecte 200 alors soumis à la force de sortie suivant l'axe longitudinal du logement.

Sur la figure 3a, le logement 120 est représenté en traits pointillés et s'étend dans l'espace intérieur du boitier 110 à partir de l'ouverture 121 pratiquée au niveau de la partie supérieure 111 du boitier. Le logement 120 est en partie délimité par le dispositif de blocage et de désengagement 130 qui comporte un organe de blocage 140, un organe de désengagement 132, un organe de déblocage 133 ainsi que la surface d'appui 131.

L'organe de blocage 140 est une pièce mobile par rapport au boitier, adaptée à être écartée ou rapprochée du logement 120. Elle comporte une surface de contact 141 orientée vers le logement 120, formée d'une première partie d'engagement 141a, située à proximité de l'ouverture 121, qui est inclinée vis-à-vis de l'axe longitudinal du logement de telle manière que l'organe de blocage 140 s'étend progressivement en direction du logement 120 à mesure qu'on s'éloigne de l'ouverture 121 du logement. La surface de contact 141 présente une deuxième partie d'appui 141b, parallèle à l'axe longitudinal du logement. La partie d'écartement 141a et la partie d'appui 141b de la surface de contact 141 sont reliées l'une à l'autre par un décrochement qui forme une surface de butée 142 d'une portion de butée 143, orientée vers la partie distale du logement vis-à-vis de l'ouverture. L'organe de blocage 140 peut s'écarter ou se rapprocher du logement, ici en étant mobile en translation suivant l'axe X. Il est néanmoins contraint en déplacement par un élément de rappel 144 qui exerce une force qui tend à rapprocher l'organe de blocage du logement, ici suivant la direction +X. Cet élément de rappel 144 est ici un ressort de compression.

Le dispositif de blocage et de désengagement 130 comporte en outre un organe de déblocage 133 adapté à déplacer l'organe de blocage 140 dans une direction opposée au logement 120, ici suivant la direction -X, de manière à repousser la portion de butée 143 hors du logement. Cet organe de déblocage 133 est ici un bouton poussoir disposé en dehors du boitier 110 et relié de manière rigide à l'organe de blocage 140.

Le dispositif de blocage et de désengagement 130 comporte en outre un organe de désengagement 132 situé en regard de la partie inférieure du logement 120 et est adapté à exercer une force de sortie sur le dispositif de collecte 200 suivant l'axe longitudinal du logement, ici suivant la direction +Z, lorsque ce dernier est inséré dans le logement.

Le dispositif de blocage et de désengagement 130 comporte également une surface d'appui 131 qui s'étend le long d'une partie du logement 120 de manière parallèle à l'axe longitudinal de celui-ci. Elle est fixée au boitier 110 sans capacité de déplacement. Cette surface d'appui peut être une surface d'une plaque fixée le long du logement. Dans le cas d'une détection optique, un capteur optique 152 est disposé du côté de la plaque d'appui opposé à la surface d'appui 131, la plaque d'appui étant formée en un matériau transparent pour les faisceaux lumineux provenant du dispositif de collecte lorsque celui-ci est inséré dans le logement.

De préférence, le dispositif de détection et d'analyse 150 comporte un capteur optique 152 situé à une distance longitudinale D2' vis-à-vis de l'ouverture 121 du logement inférieure ou égale à 2cm et de préférence inférieure ou égale à 1cm, et/ou est situé à une distance transversale D3 vis-à-vis du logement 120 inférieure ou égale à 2cm, et de préférence inférieure ou égale à 1cm. Par distance longitudinale D2', on entend une distance mesurée entre l'ouverture 121, par exemple à la surface de la paroi du boitier débouchant sur l'environnement, et le centre longitudinal suivant l'axe Z du photodétecteur 153 du capteur optique 152 ; et par distance transversale D3, on entend une distance mesurée entre le centre transversal du logement suivant l'axe X et la surface de détection du photodétecteur 153.

Sur la figure 3b qui est une vue des éléments illustrés sur la figure 3a suivant l'axe X, on voit que l'organe de blocage 140 est ici une pièce creuse en forme de U inversé, qui permet à une source de lumière située du côté opposé au logement 120 vis-à-vis de l'organe de blocage 140 de projeter des faisceaux d'illumination en direction du capteur optique sans que les faisceaux soient optiquement perturbés par l'organe de blocage.

Sur la figure 3b, et sur la figure 3c qui est une vue suivant l'axe Z des éléments illustrés sur la figure 3a, le dispositif de blocage et de désengagement 130 comporte avantageusement, dans le cas d'une détection optique, au moins un, ici deux éléments d'écartement 135 disposés de part et d'autre de la surface d'appui 131 transparente. Ces éléments d'écartement 135 permettent d'écarter le dispositif de collecte 200 de la surface d'appui 131 lors des phases d'insertion et d'éjection ou de retrait. Ils sont formés ici chacun d'une bande 135 parallèle à l'axe longitudinal du logement qui s'étend sur toute la dimension suivant Z de la surface d'appui 131, et avantageusement jusqu'à la surface de butée 142 de l'organe de blocage 140. Par ailleurs, cette bande est biseautée en ses extrémités longitudinales 135a, 135b pour éviter tout blocage du dispositif de collecte 200 lors des phases d'insertion et d'éjection ou de retrait. Pour écarter celui-ci de la surface d'appui 131 lors de son insertion et de son éjection ou retrait, ces bandes sont en saillie vis-à-vis de la surface d'appui, par exemple de quelques centaines de microns à quelques millimètres, par exemple de l'ordre de 200µm. Cela permet d'écarter les risques de frottements par le dispositif de collecte 200 sur la surface d'appui 131 transparente, frottements susceptibles d'induire une dégradation de la qualité optique de surface de la surface transparente 131, ce qui pourrait perturber les faisceaux transmis en direction du capteur optique, voire également détériorer l'assemblage du capteur optique 152 et de la plaque 134 qui subirait une contrainte de cisaillement issue de ces frottements.

Dans le cas d'un dispositif de détection optique, celui-ci comporte un photodétecteur 153 disposé en regard du logement 120, ici placé du côté de la surface d'appui 131 transparente opposé au logement, celle-ci pouvant assurer une encapsulation pour le photodétecteur 153. Le photodétecteur 153 est adapté à détecter des faisceaux lumineux provenant de l'échantillon liquide situé dans la chambre de mesure du dispositif de collecte lorsque celui-ci est inséré dans le logement. Ces faisceaux incidents sur le photodétecteur 153 peuvent être émis par l'échantillon liquide en réaction à l'illumination par une source de lumière, ou correspondre à la partie des faisceaux d'illumination transmise par l'échantillon liquide. Les faisceaux détectés peuvent être dans une gamme spectrale couvrant l'infrarouge et/ou le visible et/ou l'ultraviolet. Le photodétecteur peut être de type capteur de type CCD (pour *Charge Coupled Device,* en anglais) ou un capteur de type CMOS (pour *Complementary Metal-Oxyde Semiconductor,* en anglais), et est avantageusement matriciel dans le but d'acquérir une image de l'échantillon liquide. La source de lumière (cf. fig.1b) peut être une ou plusieurs diodes électroluminescentes, voire une diode laser par exemple à semi-conducteur, éventuellement du type VCSEL (pour *vertical-cavity surface-emitting laser,* en anglais).

Le dispositif de détection optique peut être adapté à réaliser une détection optique selon la technique dite d'imagerie sans lentille. C'est le cas notamment lorsque les analytes à détecter dans l'échantillon liquide sont des objets diffractants. Pour cela, la source de lumière 151 est choisie de manière à émettre des faisceaux d'illumination cohérents. Il peut s'agir par exemple d'une diode laser, par exemple à semi-conducteur, voire d'une diode électroluminescente munie d'un diaphragme permettant d'augmenter la cohérence du rayonnement émis ou encore d'une diode électroluminescente dont les dimensions sont suffisamment réduites pour ne pas avoir à utiliser de diaphragme, par exemple le diamètre de la diode étant inférieur au dixième de la distance séparant la diode du logement. Le photodétecteur 153 est matriciel pour ainsi acquérir des images du rayonnement transmis et/ou diffracté par les analytes présents dans l'échantillon liquide. Il est placé à une distance du logement 120, par exemple à une position centrale du logement suivant l'axe X, comprise entre 100µm et quelques centimètres, par exemple inférieure ou égale à 1cm, et avantageusement comprise entre 100µm et 2mm. La faible distance entre la chambre de mesure 221 présente dans le logement et le photodétecteur 153 permet de limiter les phénomènes d'interférence entre les figures de diffraction lorsque la chambre de mesure est éclairée. On peut éviter le recours à une optique de grossissement qui serait placée entre le logement et le photodétecteur. Un réseau de microlentilles, chacune étant placée devant un pixel du photodétecteur, peut être prévu pour améliorer la collecte optique de chaque pixel du photodétecteur, sans pour autant qu'une fonction de grossissement soit assurée.

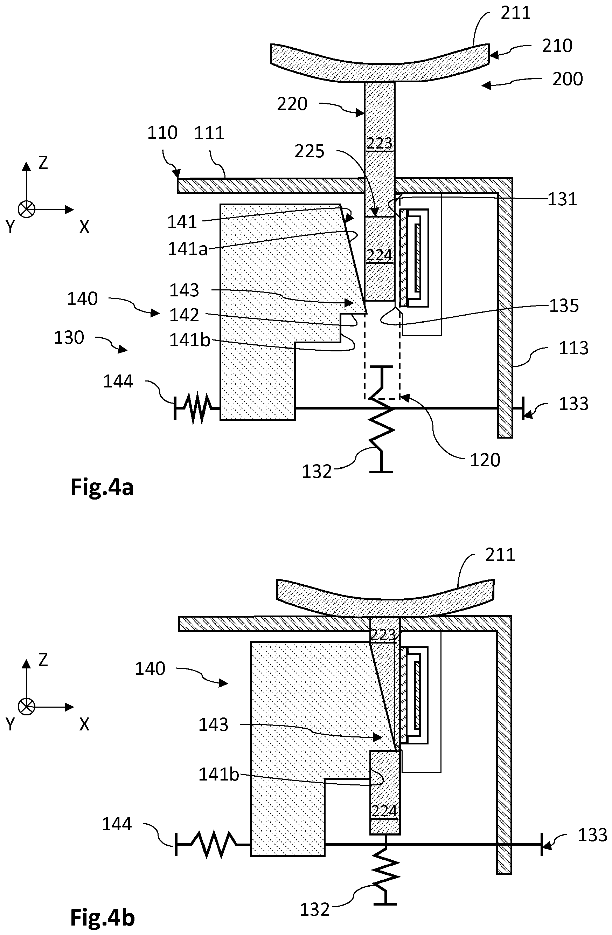

Le procédé d'analyse est maintenant décrit en référence aux figures 4a et 4b qui illustrent le dispositif de collecte en cours d'insertion dans l'appareil d'analyse (figure 4a) et lorsqu'il se trouve en position de blocage (figure 4b).

Comme le montre la figure 4a, le dispositif de collecte 200 est inséré dans le logement 120 de l'appareil d'analyse 100. Au cours de cette phase d'insertion, le talon 224 vient au contact de la partie d'engagement 141a de la surface de contact 141 de l'organe de blocage 140, et à mesure qu'il est introduit dans le logement, il repousse progressivement l'organe de blocage 140 dans la direction -X, ce qui conduit à la mise en charge du ressort 144. Puis, il vient au contact de l'organe de désengagement 132, ici un ressort de compression, et entraîne la mise en charge du ressort, ce qui se traduit par une force de sortie appliquée par l'organe de désengagement 132 sur le corps principal 220. Lorsqu'on souhaite éviter les frottements du dispositif de collecte 200 sur la surface d'appui 131 lors des phases d'insertion et d'éjection ou de retrait, les éléments d'écartement 135 sont prévus pour assurer l'écartement du corps principal 220 vis-à-vis de la surface d'appui 131 par contact entre les bandes 135 et le talon 224, avant l'engagement de la portion de butée 143 de l'organe de blocage vis-à-vis de la portion d'appui 225 du corps principal 220.

Comme le montre la figure 4b, la position de blocage est obtenue lorsque les portions d'appui 225 du corps principal 220 (illustrés sur la figure 4a) dépassent la portion de butée 143 de l'organe de blocage 140 suivant la direction -Z. Le ressort de rappel 144 entraîne alors le déplacement de l'organe de blocage 140 en direction du logement de manière à provoquer l'engagement de la butée, c'est-à-dire la mise au contact de la portion de butée 143 avec la portion d'appui 225. Par la même occasion, la partie d'appui 141b de la surface de contact 141 exerce une force sur le talon 224 du dispositif de collecte suivant la direction +X de sorte que celui-ci vient au contact de la surface d'appui 131. Cela est notamment rendu possible du fait que le talon 224 n'est plus au contact des éléments d'écartement 135 et que la partie centrale 223 du corps principal 220, dont la largeur suivant l'axe Y est inférieure à celle du talon 224 et à l'espacement entre les éléments d'écartement 135, n'est pas repoussée par ces derniers.

Ainsi, le dispositif de collecte 200 est bloqué suivant l'axe Z par l'action conjointe de l'organe de désengagement 132 qui exerce sur celui-ci une force suivant la direction +Z et par la portion de butée 143 de l'organe de blocage 140 qui exerce une force de réaction suivant la direction -Z sur la portion d'appui 225 du dispositif de collecte 200. Par ailleurs, le dispositif de collecte 200 est bloqué suivant l'axe X par le contact avec la surface d'appui 131 par la force exercée suivant la direction +X par la partie d'appui 141b de la surface de contact 141 de l'organe de blocage 140 contre le talon 224.

Le dispositif de collecte est ainsi bloqué dans le logement et positionné de manière parfaitement contrôlée vis-à-vis de la surface d'appui, ce qui permet d'une part d'éviter tout risque de déplacement involontaire de celui-ci lors de l'étape ultérieure de détection, et permet ainsi d'améliorer la précision et la fiabilité de la détection lorsqu'elle repose sur une technique optique, en particulier lorsqu'il s'agit de la technique d'imagerie sans lentille décrite précédemment.

Le dépôt de l'échantillon liquide est ensuite effectué. Pour cela, un volume de liquide est déposé sur la surface de réception 211 du dispositif de collecte, par exemple une goutte de sang présente au niveau du doigt d'une personne. Comme mentionné précédemment, la surface de réception peut, à cette étape, être orientée de manière sensiblement orthogonale à l'axe de la gravité. Dans le cas où la partie de collecte est en contact avec le boitier lors de l'appui par un utilisateur sur la surface de réception, on minimise les contraintes mécaniques subies par le dispositif de collecte et transmises dans la paroi du boitier. De plus, cet agencement de la surface de réception vis-à-vis de la chambre de mesure permet de n'avoir à déposer qu'un volume limité d'échantillon liquide, la distance séparant l'orifice de collecte de la chambre de mesure et celle séparant l'ouverture du logement vis-à-vis du capteur optique est minimisée. Lors de cette phase de dépôt, l'appareil de mesure 100 peut être maintenu à la main par un utilisateur.

L'échantillon liquide migre, par exemple par capillarité, à partir de l'orifice de collecte en direction de la chambre de mesure, des réactifs (séchés ou lyophilisés) pouvant être présents dans la chambre de mesure 221 et/ou dans le conduit fluidique 222 (voir figure 1b ou 2a). Il s'en suit dans ce cas une réaction chimique dont un paramètre est détecté puis analysé. La détection peut, à titre d'exemple, être colorimétrique, le dispositif de détection optique mesurant alors par photométrie la couleur finale de l'échantillon lors de la réaction, l'intensité de la couleur étant proportionnelle à la concentration en analyte présent dans l'échantillon. Comme décrit précédemment, il est possible d'effectuer une détection optique particulièrement optimisée par imagerie sans lentille, du fait notamment du positionnement contrôlé et rapproché du capteur optique vis-à-vis de la chambre de mesure par l'intermédiaire de la surface d'appui contre laquelle le corps principal du dispositif de collecte est plaqué. Les données brutes issues du dispositif de détection sont transmises à l'unité d'analyse qui procède au traitement des informations mesurées et affiche sur l'écran les résultats à l'intention de l'utilisateur.

Après détection et éventuellement analyse de l'échantillon liquide, l'éjection ou le retrait du dispositif de collecte 200 vis-à-vis de l'appareil d'analyse 100 peut être effectué par l'utilisateur. Pour cela, il actionne l'organe de déblocage 133 qui entraîne le déplacement de l'organe de blocage 140 jusqu'à provoquer une libération de la butée, c'est-à-dire une mise hors contact mécanique entre la portion de butée 143 et la portion d'appui 225 du dispositif de collecte. La force de rappel appliquée par l'organe de désengagement 132, ici le ressort chargé, sur le dispositif de collecte 200 entraîne la sortie de préférence partielle, autrement dit le retrait partiel, de ce dernier vis-à-vis du logement 120. Lorsque les éléments d'écartement 135 sont présents, le talon 224 vient au contact des éléments d'écartement de sorte que le dispositif de collecte n'est plus au contact de la surface d'appui 131 transparente pendant toute la phase de déplacement. La force de sortie entraîne donc ici le retrait partiel du dispositif de collecte qui se retrouve partiellement logé dans le logement 120 dans une position débloquée. Il est alors possible de retourner l'appareil d'analyse pour que le dispositif de collecte sorte entièrement du logement par gravité, et vienne éventuellement tomber dans un récipient prévu à cet effet. Cela permet d'éviter d'avoir à manipuler le dispositif de collecte dont la surface de réception peut contenir des traces d'échantillon liquide. Bien entendu, en variante, la force de rappel de l'organe de désengagement 132 peut être suffisante pour entraîner l'éjection totale du dispositif de collecte 200 hors du logement 120.

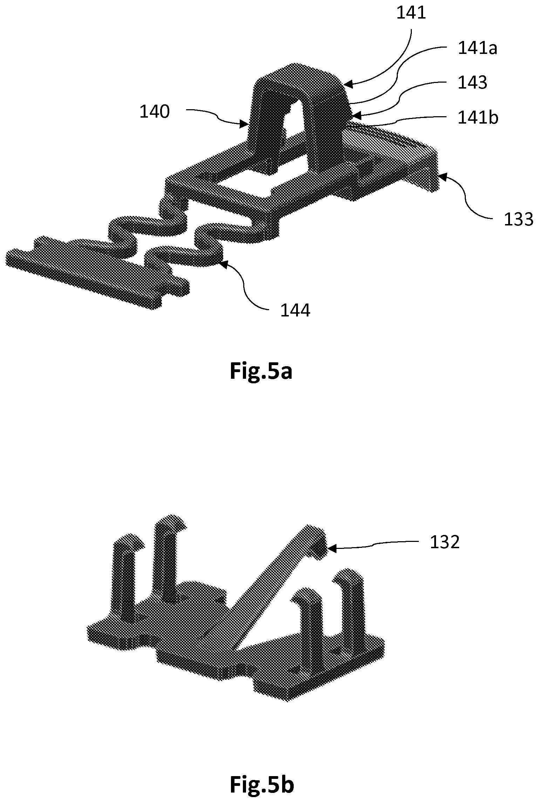

Les figures 5a et 5b sont des vues en perspective d'un exemple d'une partie du dispositif de blocage et de désengagement 130, en particulier l'organe de blocage (fig.5a) et l'organe de désengagement (fig.5b).

Sur la figure 5a est illustrée une pièce mécanique formée d'un seul tenant comportant l'organe de blocage 140 creux en forme de pont, qui repose sur une base qui s'étend de manière rigide d'un côté jusqu'à une partie formant l'organe de déblocage 133, c'est-à-dire ici un bouton poussoir, et d'un côté opposé jusqu'à une partie flexible formant l'élément de rappel 144 sous forme d'un ressort de compression.

Sur la figure 5b est illustrée une pièce mécanique formant l'organe de désengagement 132. Cette pièce comporte une lame flexible inclinée de manière à ce qu'elle puisse exercer une force de sortie lorsque le dispositif de collecte est inséré dans le logement et en position de blocage. Cette pièce est destinée à être engagée sous l'organe de blocage 140 au niveau de la partie ajourée de la base illustrée sur la figure 5a.

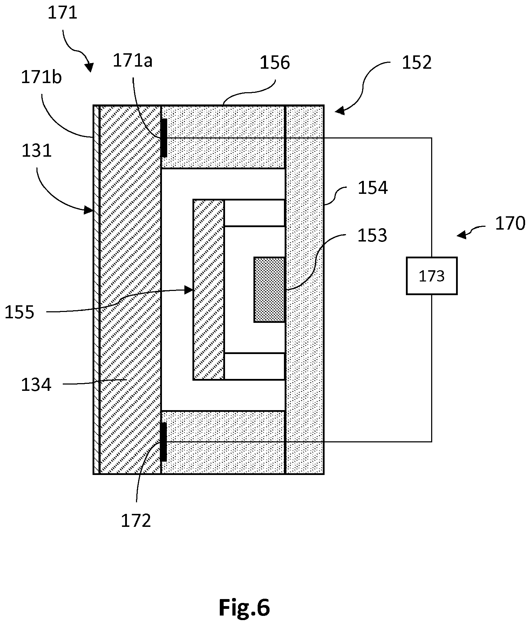

La figure 6 est une vue schématique en coupe d'un exemple de capteur optique 152 muni d'un dispositif de chauffage 170 permettant d'assurer une régulation thermique de la surface d'appui 131 et ainsi de la partie centrale du corps principal du dispositif de collecte contenant la chambre de mesure.

Le capteur optique 152 comporte ici un photodétecteur 153 qui repose sur une carte électronique 154, par exemple un circuit imprimé (PCB, pour *Printed Circuit Board,* en anglais), par l'intermédiaire d'un boitier céramique (non représenté), pour alimenter électriquement le photodétecteur 153 et transmettre les signaux de détection en direction de l'unité d'analyse. Le photodétecteur est ici encapsulé dans un capot de protection secondaire 155 comportant une plaque transparente aux faisceaux incidents. La surface d'appui 131, ici la face orientée vers le logement de la plaque 134, repose sur une seconde carte électronique 156 périphérique qui comporte une partie d'un dispositif chauffant 170.

Le dispositif de chauffage 170 comporte au moins un élément chauffant 171, et de préférence au moins une sonde de température 172 et une unité de régulation thermique 173, l'élément chauffant et la sonde de température étant électriquement reliés à l'unité de régulation thermique.

L'élément chauffant 171 est adapté à émettre de la chaleur, par exemple par effet Joule, et est placé de manière à pouvoir transmettre ce flux thermique à la partie centrale du corps principal lorsqu'elle est au contact de la surface d'appui 131. Il peut s'agir d'une piste électriquement conductrice 171a, par exemple métallique, apte à dégager de la chaleur lorsqu'elle est parcourue par un courant électrique, associée à une couche thermiquement conductrice 171b et de préférence transparente. La couche thermiquement conductrice 171b peut ainsi être située au niveau de la surface de la plaque 134 orientée vers le logement et ainsi former la surface d'appui 131, et/ou être disposé sur sa face opposée. Elle peut être une couche en un matériau transparent, par exemple du silicium amorphe voire de l'oxyde d'indium-étain (ITO, pour *Indium Tin Oxide,* en anglais) déposé par sérigraphie ou par pulvérisation sur la face de la plaque. Cette couche 172b assure la propagation de la chaleur émise par la piste métallique 171a, celle-ci étant par exemple située à l'interface entre la carte périphérique 156 et la plaque d'appui 134. Elle est électriquement connectée à une source de courant, par exemple par l'intermédiaire du plot périphérique et de la carte électronique, cette dernière étant alors connectée à l'unité de régulation 173.

En variante, l'élément chauffant peut comporter, outre la piste électriquement conductrice, la plaque d'appui 134 elle-même. Dans ce cas, la plaque est réalisée en un matériau thermiquement conducteur et de préférence optiquement transparent, comme par exemple du saphir. La couche 171b peut alors être omise.

Une sonde 172 apte à mesurer la température est ici prévue et placée de préférence au plus près de la surface d'appui 131. Elle est reliée électriquement à l'unité de régulation 173 pour lui transmettre une valeur de température mesurée.

L'unité de régulation 173 est électriquement reliée à la piste métallique 171a et à la sonde de température 172. Elle envoie à une source de courant un signal de consigne qui se traduit par une valeur de courant électrique parcourant la piste 171a et ainsi par une valeur de température cible. Cette valeur de température cible peut être corrigée en fonction de la valeur de la température mesurée par la sonde 172.

Ainsi, le dispositif de chauffage 170 permet d'assurer la régulation thermique de l'échantillon liquide lorsque le corps principal 220 du dispositif de collecte est au contact de la surface d'appui 131. La surface d'appui présente ainsi l'avantage supplémentaire, outre le fait de permettre un positionnement contrôlé de la chambre de mesure vis-à-vis du photodétecteur, de permettre de réguler thermiquement la chambre de mesure.

Des modes de réalisation particuliers viennent d'être décrits. Différentes variantes et modifications apparaîtront à l'homme du métier.

Ainsi, le dispositif de détection peut assurer, une détection électrochimique d'une ou plusieurs propriétés de l'échantillon liquide, de manière alternative ou en complément d'une détection optique. Pour cela, la chambre de mesure peut être munie d'une ou plusieurs électrodes. Lorsque le dispositif de collecte est inséré dans l'appareil d'analyse, ces électrodes peuvent venir au contact de plots de reprise de contact électrique, par exemple placés au niveau de la surface d'appui, ces plots étant électriquement connectés à une source de tension ou de courant. Ainsi, une différence de potentiel ou de courant peut être appliquée aux électrodes et une propriété électrochimique du liquide peut être détectée puis analysée.

Par ailleurs, on a décrit un logement dont l'ouverture est située au niveau de la paroi supérieure du boitier. Cependant, elle peut être située au niveau de la paroi latérale, notamment lorsque l'appareil d'analyse est destiné à coopérer avec un dispositif de collecte dont la partie de collecte est parallèle est sensiblement coplanaire avec le corps principal contenant la chambre de mesure.

## Revendications

1. Appareil d'analyse (100) d'un échantillon liquide adapté à coopérer avec un dispositif de collecte (200) dudit échantillon liquide, comprenant :
- un boitier (110) délimitant un espace intérieur et comprenant une ouverture (121) traversante débouchant sur un logement (120) adapté à recevoir un corps principal (220) du dispositif de collecte (200) et disposé dans l'espace intérieur en s'étendant longitudinalement à partir de l'ouverture (121) suivant un axe longitudinal dit d'insertion du dispositif de collecte (200),
- un dispositif de détection et d'analyse (150) d'au moins un paramètre représentatif de l'échantillon liquide,
**caractérisé en ce qu'**il comporte un dispositif de blocage et de désengagement (130) adapté à assurer le maintien dans une position de blocage du dispositif de collecte lorsque le corps principal (220) est inséré à l'intérieur du logement (120), et comprenant :
- un organe de désengagement (132) adapté à exercer une force de sortie sur le corps principal (220) du dispositif de collecte, dans une direction sensiblement parallèle à l'axe longitudinal du logement et orientée vers l'ouverture (121) du logement ;
- un organe de blocage (140) comportant au moins une portion de butée (143) adaptée à être au contact d'une portion d'appui (225) du corps principal (220) du dispositif de collecte, de manière à immobiliser le dispositif de collecte (200) alors soumis à la force de sortie suivant l'axe longitudinal du logement.

2. Appareil d'analyse (100) selon la revendication 1, comportant une surface d'appui (131), fixe par rapport au logement (120), et disposée de manière à délimiter en partie celui-ci en s'étendant de manière parallèle à l'axe longitudinal du logement et de manière à pouvoir être au contact d'une partie du corps principal (220) contenant une chambre fluidique de mesure (221) du dispositif de collecte lorsque celui-ci est inséré dans le logement (120).

3. Appareil d'analyse (100) selon la revendication 1 ou 2, dans lequel l'organe de blocage (140) est mobile vis-à-vis du logement (120) de manière sensiblement transversale à l'axe longitudinal de celui-ci et adapté à exercer une force d'appui sur le corps principal (220) en direction de la surface d'appui (131) lorsque celui-ci est inséré dans le logement.

4. Appareil d'analyse (100) selon l'une quelconque des revendications 1 à 3, dans lequel l'organe de blocage (140) comporte une surface de contact (141) orientée vers le logement (120) présentant une partie d'écartement (141a) proximale vis-à-vis de l'ouverture (121) du logement et inclinée vis-à-vis de l'axe longitudinal du logement, et une partie d'appui (141b) distale, entre lesquelles se situe la portion de butée (143).

5. Appareil d'analyse (1) selon l'une quelconque des revendications 1 à 4, dans lequel le dispositif de blocage et de désengagement (130) comporte un organe de déblocage (133) dont l'actionnement est adapté à provoquer la mise en mouvement de l'organe de blocage (140) de manière à provoquer un échappement de la butée entre la portion de butée (143) de celui-ci et la portion d'appui (225) du dispositif de collecte, conduisant à un retrait du corps principal (220) vis-à-vis du logement sous l'effet de la force de sortie.

6. Appareil d'analyse (100) selon l'une quelconque des revendications 2 à 5 dépendant de la revendication 2, comportant au moins un élément d'écartement (135) disposé longitudinalement le long d'une bordure de la surface d'appui (131) et en saillie vis-à-vis de celle-ci en direction du logement, dont les extrémités longitudinales (135a, 135b) sont biseautées, de manière à écarter le corps principal (220) vis-à-vis de la surface d'appui (131) lors du déplacement de celui-ci dans le logement selon l'axe longitudinal.

7. Appareil d'analyse (100) selon l'une quelconque des revendications 2 à 6 dépendant de la revendication 2, dans lequel le dispositif de détection et d'analyse (150) comporte un capteur optique (152) situé d'un côté de la surface d'appui (131) opposé au logement (120), la surface d'appui (131) étant transparente à des faisceaux lumineux provenant de la chambre de mesure (221) lorsque le corps principal (220) du dispositif de collecte est inséré dans le logement (120).

8. Appareil d'analyse (100) selon l'une quelconque des revendications 1 à 7, dans lequel le dispositif de détection et d'analyse (150) comporte un capteur optique (152) situé à une distance longitudinale (D2') vis-à-vis de l'ouverture (121) du logement inférieure ou égale à 2cm et/ou à une distance transversale (D3) vis-à-vis du logement (120) inférieure ou égale à 2cm.

9. Appareil d'analyse (100) selon l'une quelconque des revendications 2 à 8 dépendant de la revendication 2, dans lequel un dispositif de chauffage (170) comprend un élément chauffant (171) adapté à transmettre de la chaleur au corps principal (220) par l'intermédiaire de la surface d'appui (131) lorsque ce dernier est inséré dans le logement et au contact de la surface d'appui.

10. Appareil d'analyse (100) selon l'une quelconque des revendications 1 à 9, dans lequel le dispositif de détection et d'analyse (150) comporte au moins une source de lumière (151) et un capteur optique (152) disposés de part et d'autre du logement (120) suivant un axe d'éclairement du corps principal (221) du dispositif de collecte lorsqu'il est inséré dans le logement, l'organe de blocage (140) comportant une partie creuse positionnée entre la source de lumière (151) et le capteur optique (152) permettant la propagation des faisceaux d'illumination suivant l'axe d'éclairement au travers de ladite partie creuse.

11. Appareil d'analyse (100) selon l'une quelconque des revendications 1 à 10, le boitier comportant une paroi supérieure (111) et une paroi inférieure (112) reliées l'une à l'autre par une paroi latérale (113) présentant une largeur entre lesdites parois inférieure aux dimensions des parois supérieure et inférieure (111, 112), l'ouverture (121) du logement est située au niveau de la paroi supérieure (111) du boitier (110) et le logement s'étend de manière sensiblement orthogonale à celle-ci en direction de la paroi inférieure.

12. Appareil d'analyse (100) selon la revendication précédente, dans lequel la paroi supérieure (111) du boitier comprend un écran d'affichage (114).

13. Système d'analyse (1) d'un échantillon liquide, comportant un appareil d'analyse (100) selon l'une quelconque des revendications précédentes, **caractérisé en ce qu'**il comporte en outre un dispositif de collecte (200) dudit l'échantillon liquide, comprenant :
- un corps principal (220) s'étendant suivant un axe longitudinal et comportant une chambre fluidique (221) de mesure, apte à être inséré longitudinalement dans le logement (120) suivant l'axe longitudinal d'insertion de celui-ci ;
- une surface de réception (211), destinée à être située hors du logement (120) pour recevoir l'échantillon liquide lorsque le corps principal (220) est inséré dans le logement (120), assemblée au corps principal (220) et comprenant un orifice de collecte (212) communiquant fluidiquement avec la chambre de mesure (221).

14. Système d'analyse (1) selon la revendication 13, dans lequel la surface de réception (211) s'étend de manière sensiblement orthogonale à l'axe longitudinal du corps principal (220).

15. Système d'analyse (1) selon la revendication 14, dans lequel le logement (120) de l'appareil d'analyse (100) et le dispositif de collecte (200) sont dimensionnés l'un et l'autre de sorte que, lorsque le corps principal (220) du dispositif de collecte est inséré dans le logement, la distance (D1) entre l'orifice de collecte (212) et la chambre de mesure (221) est approximativement égale à la distance (D2) entre l'ouverture du logement (121) et la chambre de mesure (221), et est de préférence inférieure ou égale à 2cm.

16. Système d'analyse (1) selon la revendication 14 ou 15, dans lequel le dispositif de collecte (200) comporte une partie de collecte (210) assemblée au corps principal (220) et dont une surface forme la surface de réception (211), cette partie de collecte (210) étant au contact d'une paroi du boitier (110) en bordure de l'ouverture (121) du logement lorsque le corps principal (220) du dispositif de collecte est inséré dans le logement (120).

17. Système d'analyse (1) selon l'une quelconque des revendications 13 à 16, dans lequel la portion d'appui (225) du corps principal (220) du dispositif de collecte est formée par un épaulement situé entre une première partie dite centrale (223) contenant une chambre de mesure (221) et une extrémité distale du corps principal (220) vis-à-vis de la chambre de mesure (221).

## Patentansprüche

1. Analysevorrichtung (100) einer flüssigen Probe, die dazu ausgelegt ist, mit einer Sammeleinrichtung (200) der flüssigen Probe zusammenzuwirken, beinhaltend:
- ein Gehäuse (110), das einen Innenraum abgrenzt und eine Durchgangsöffnung (121) beinhaltet, die in eine Kammer (120) führt, welche dazu ausgelegt ist, einen Grundkörper (220) der Sammeleinrichtung (200) aufzunehmen, und in dem Innenraum angeordnet ist, indem sie sich von der Öffnung (121) entlang einer als Einführungsachse bezeichneten Längsachse der Sammeleinrichtung (200) längs erstreckt,
- eine Einrichtung (150) zum Detektieren und Analysieren mindestens eines repräsentativen Parameters der flüssigen Probe,
**dadurch gekennzeichnet, dass** sie eine Einrichtung zum Blockieren und Lösen (130) umfasst, die dazu ausgelegt ist, das Halten der Sammeleinrichtung in einer Blockierposition zu gewährleisten, wenn der Grundkörper (220) in das Innere der Kammer (120) eingeführt ist, und beinhaltend:
- ein Löseorgan (132), das dazu ausgelegt ist, in einer Richtung, die zu der Längsachse der Kammer im Wesentlichen parallel ist und zu der Öffnung (121) der Kammer hin gerichtet ist, eine Austrittskraft auf den Grundkörper (220) der Sammeleinrichtung auszuüben;
- ein Blockierorgan (140), das mindestens einen Anschlagabschnitt (143) umfasst, der dazu ausgelegt ist, mit einem Anlageabschnitt (225) des Grundkörpers (220) der Sammeleinrichtung in Kontakt zu sein, um die Sammeleinrichtung (200), die dann gemäß der Längsachse der Kammer der Austrittskraft ausgesetzt ist, zu immobilisieren.

2. Analysevorrichtung (100) nach Anspruch 1, umfassend eine Anlagefläche (131), die in Bezug auf die Kammer (120) feststehend ist und so angeordnet ist, dass sie diese teilweise begrenzt, indem sie sich parallel zu der Längsachse der Kammer erstreckt, und dass sie mit einem Teil des Grundkörpers (220), der einen Messfluidraum (221) der Sammeleinrichtung enthält, in Kontakt sein kann, wenn dieser in die Kammer (120) eingeführt ist.

3. Analysevorrichtung (100) nach Anspruch 1 oder 2, wobei das Blockierorgan (140) in Bezug auf die Kammer (120) zu der Längsachse derselben im Wesentlichen quer bewegbar ist und dazu ausgelegt ist, eine Anlagekraft in Richtung der Anlagefläche (131) auf den Grundkörper (220) auszuüben, wenn dieser in die Kammer eingeführt ist.

4. Analysevorrichtung (100) nach einem der Ansprüche 1 bis 3, wobei das Blockierorgan (140) eine zu der Kammer (120) hin gerichtete Kontaktfläche (141) umfasst, die einen in Bezug auf die Öffnung (121) der Kammer (141a) proximalen und in Bezug auf die Längsachse der Kammer geneigten Abstandsteil und einen distalen Anlageteil (141b) aufweist, zwischen denen sich der Anschlagabschnitt (143) befindet.

5. Analysevorrichtung (1) nach einem der Ansprüche 1 bis 4, wobei die Blockier- und Löseeinrichtung (130) ein Deblockierorgan (133) umfasst, dessen Betätigung dazu ausgelegt ist, das Bewegen des Blockierorgans (140) zu bewirken, um ein Freisetzen des Anschlags zwischen dem Anschlagabschnitt (143) desselben und dem Anlageabschnitt (225) der Sammeleinrichtung zu bewirken, was unter der Einwirkung der Austrittskraft zu einem Rückzug des Grundkörpers (220) in Bezug auf die Kammer führt.

6. Analysevorrichtung (100) nach einem der Ansprüche 2 bis 5 in Abhängigkeit von Anspruch 2, umfassend mindestens ein Abstandselement (135), das längs entlang eines Rands der Anlagefläche (131) angeordnet ist und in Bezug auf diese in Richtung der Kammer vorspringt, dessen Längsenden (135a, 135b) abgeschrägt sind, um den Grundkörper (220) in Bezug auf die Anlagefläche (131) auf Abstand zu halten, wenn sich dieser gemäß der Längsachse in der Kammer bewegt.

7. Analysevorrichtung (100) nach einem der Ansprüche 2 bis 6 in Abhängigkeit von Anspruch 2, wobei die Detektions- und Analyseeinrichtung (150) einen optischen Sensor (152) umfasst, der sich auf einer Seite der Anlagefläche (131), die der Kammer (120) entgegengesetzt ist, befindet, wobei die Anlagefläche (131) für Lichtstrahlen, die, wenn der Grundkörper (220) der Sammeleinrichtung in die Kammer (120) eingeführt ist, aus dem Messraum (221) austreten, transparent ist.

8. Analysevorrichtung (100) nach einem der Ansprüche 1 bis 7, wobei die Detektions- und Analyseeinrichtung (150) einen optischen Sensor (152) umfasst, der in Bezug auf die Öffnung (121) der Kammer mit einem Längsabstand (D2') von kleiner oder gleich 2 cm und/oder in Bezug auf die Kammer (120) mit einem Querabstand (D3) von kleiner oder gleich 2 cm eingerichtet ist.

9. Analysevorrichtung (100) nach einem der Ansprüche 2 bis 8 in Abhängigkeit von Anspruch 2, wobei eine Heizeinrichtung (170) ein Heizelement (171) beinhaltet, das dazu ausgelegt ist, mittels der Anlagefläche (131) Wärme an den Grundkörper (220) zu übertragen, wenn dieser in die Kammer eingeführt und mit der Anlagefläche in Kontakt ist.

10. Analysevorrichtung (100) nach einem der Ansprüche 1 bis 9, wobei die Detektions- und Analyseeinrichtung (150) mindestens eine Lichtquelle (151) und einen optischen Sensor (152) umfasst, die gemäß einer Beleuchtungsachse des Grundkörpers (221) der Sammeleinrichtung, wenn dieser in die Kammer eingeführt ist, auf beiden Seiten der Kammer (120) angeordnet sind, wobei das Blockierorgan (140) einen hohlen Teil umfasst, der zwischen der Lichtquelle (151) und dem optischen Sensor (152) positioniert ist und die Ausbreitung der Lichtstrahlen entlang der Beleuchtungsachse durch den hohlen Teil gestattet.

11. Analysevorrichtung (100) nach einem der Ansprüche 1 bis 10, wobei das Gehäuse eine obere Wand (111) und eine untere Wand (112) umfasst, die durch eine Seitenwand (113), die eine Breite zwischen diesen Wänden kleiner als die Maße der oberen und der unteren Wand (111, 112) aufweist, miteinander verbunden sind, wobei sich die Öffnung (121) der Kammer im Bereich der oberen Wand (111) des Gehäuses (110) befindet und sich die Kammer im Wesentlichen orthogonal zu dieser in Richtung der unteren Wand erstreckt.

12. Analysevorrichtung (100) nach dem vorhergehenden Anspruch, wobei die obere Wand (111) des Gehäuses einen Bildschirm (114) beinhaltet.

13. Analysesystem (1) einer flüssigen Probe, umfassend eine Analysevorrichtung (100) nach einem der vorhergehenden Ansprüche, **dadurch gekennzeichnet, dass** es ferner eine Einrichtung (200) zum Sammeln der flüssigen Probe umfasst, beinhaltend:
- einen Grundkörper (220), der sich gemäß einer Längsachse erstreckt und einen Messfluidraum (221) umfasst und der fähig ist, gemäß der Einführungslängsachse der Kammer (120) längs in dieselbe eingeführt zu werden;
- eine Aufnahmefläche (211), die dazu bestimmt ist, sich außerhalb der Kammer (120) zu befinden, um die flüssige Probe aufzunehmen, wenn der Grundkörper (220) in die Kammer (120) eingeführt ist, die an dem Grundkörper (220) befestigt ist und eine Sammelöffnung (212) beinhaltet, die fluidisch mit dem Messraum (221) kommuniziert.

14. Analysesystem (1) nach Anspruch 13, wobei sich die Aufnahmefläche (211) im Wesentlichen orthogonal zu der Längsachse des Grundkörpers (220) erstreckt.

15. Analysesystem (1) nach Anspruch 14, wobei die Kammer (120) der Analysevorrichtung (100) und die Sammeleinrichtung (200) jeweils so bemessen sind, dass, wenn der Grundkörper (220) der Sammeleinrichtung in die Kammer eingeführt ist, der Abstand (D1) zwischen der Sammelöffnung (212) und dem Messraum (221) ungefähr gleich dem Abstand (D2) zwischen der Öffnung der Kammer (121) und dem Messraum (221) ist und vorzugsweise kleiner oder gleich 2 cm ist.

16. Analysesystem (1) nach Anspruch 14 oder 15, wobei die Sammeleinrichtung (200) einen Sammelteil (210) umfasst, der an dem Grundkörper (220) befestigt ist und von dem eine Fläche die Aufnahmefläche (211) bildet, wobei dieser Sammelteil (210) an dem Rand der Öffnung (121) der Kammer mit einer Wand des Gehäuses (110) in Kontakt ist, wenn der Grundkörper (220) der Sammeleinrichtung in die Kammer (120) eingeführt ist.

17. Analysesystem (1) nach einem der Ansprüche 13 bis 16, wobei der Anlageabschnitt (225) des Grundkörpers (220) der Sammeleinrichtung durch einen Absatz gebildet ist, der sich zwischen einem sogenannten zentralen Teil (223), der einen Messraum (221) enthält, und einem in Bezug auf den Messraum (221) distalen Ende des Grundkörpers (220) befindet.

## Claims

1. Apparatus (100) for analyzing a liquid sample adapted to cooperate with a device (200) for collecting said liquid sample, comprising:
- a housing (110) delimiting an interior space and comprising a through-opening (121) leading into a recess (120) adapted to receive a main body (220) of the collection device (200) disposed in the interior space and extending longitudinally from the opening (121) along a longitudinal so-called insertion axis of the collection device (200),
- a device (150) for detection and analysis of at least one representative parameter of the liquid sample,
**characterized in that** it includes a locking and releasing device (130) adapted to retain the collection device in a locking position when the main body (220) is inserted in the recess (120), and comprising:
- a releasing member (132) adapted to exert an ejection force on the main body (220) of the collection device in a direction substantially parallel to the longitudinal axis of the recess and oriented toward the opening (121) of the recess;
- a locking member (140) including at least one abutment portion (143) adapted to be in contact with a bearing portion (225) of the main body (220) of the collection device so as to immobilize the collection device (200) then subjected to the ejection force along the longitudinal axis of the recess.

2. Analysis apparatus (100) according to Claim 1, including a bearing surface (131) fixed relative to the recess (120) and disposed so as to delimit the latter in part, extending in a parallel manner to the longitudinal axis of the recess, and so as to be able to be in contact with a portion of the main body (220) containing a fluidic measurement chamber (221) of the collection device when the latter is inserted in the recess (120).

3. Analysis apparatus (100) according to Claim 1 or 2, wherein the locking member (140) is mobile relative to the recess (120) in a manner substantially transverse to the longitudinal axis thereof and adapted to exert a bearing force on the main body (220) in the direction of the bearing surface (131) when the latter body is inserted in the recess.

4. Analysis apparatus (100) according to any one of Claims 1 to 3, wherein the locking member (140) includes a contact surface (141) oriented toward the recess (120) having a spacing part (141a) proximal to the opening (121) of the recess and inclined relative to the longitudinal axis of the recess and a distal bearing part (141b), between which parts the abutment portion (143) is located.

5. Analysis apparatus (1) according to any one of Claims 1 to 4, wherein the locking and releasing device (130) includes an unlocking member (133) actuation of which is adapted to cause the locking member (140) to be moved in such a manner as to bring about an escape from the abutment between the abutment portion (143) thereof and the bearing portion (225) of the collection device, leading to withdrawal of the main body (220) relative to the recess because of the effect of the ejection force.

6. Analysis apparatus (100) according to any one of Claims 2 to 5 dependent on Claim 2, including at least one spacing element (135) disposed longitudinally along an edge of the bearing surface (131) and projecting relative thereto in the direction of the recess and the longitudinal ends (135a, 135b) of which are beveled so as to move the main body (220) away from the bearing surface (131) when it moves in the recess along the longitudinal axis.

7. Analysis apparatus (100) according to any one of Claims 2 to 6 dependent on Claim 2, wherein the detection and analysis device (150) includes an optical sensor (152) located on the opposite side of the bearing surface (131) to the recess (120), the bearing surface (131) being transparent to light beams emanating from the measuring chamber (221) when the main body (220) of the collection device is inserted in the recess (120).

8. Analysis apparatus (100) according to any one of Claims 1 to 7, wherein the detection and analysis device (150) includes an optical sensor (152) situated at a longitudinal distance (D2') from the opening (121) of the recess less than or equal to 2 cm and/or at a transverse distance (D3) from the recess (120) less than or equal to 2 cm.

9. Analysis apparatus (100) according to any one of Claims 2 to 8 dependent on Claim 2, wherein a heating device (170) comprises a heating element (171) adapted to transmit heat to the main body (220) via the bearing surface (131) when it is inserted in the recess and in contact with the bearing surface.

10. Analysis apparatus (100) according to any one of Claims 1 to 9, wherein the detection and analysis device (150) includes at least one light source (151) and an optical sensor (152) disposed on respective opposite sides of the recess (120) along an illumination axis of the main body (221) of the collection device when it is inserted in the recess, the locking member (140) including a hollow part positioned between the light source (151) and the optical sensor (152) enabling propagation of the illumination beams along the illumination axis through said hollow part.

11. Analysis apparatus (100) according to any one of Claims 1 to 10, the housing including an upper wall (111) and a lower wall (112) connected to each other by a lateral wall (113) having a width between said walls less than the dimensions of the upper and lower walls (111, 112), the opening (121) of the housing is located at the level of the upper wall (111) of the housing (110) and the recess extends in a substantially orthogonal manner to the latter wall in the direction of the lower wall (110).

12. Analysis apparatus (100) according to the preceding claim, wherein the upper wall (111) of the housing comprises a display screen (114).

13. System (1) for analyzing a liquid sample, including an analysis apparatus (100) according to any one of the preceding claims, **characterized in that** it further includes a device (200) for collecting said liquid sample comprising:
- a main body (220) extending along a longitudinal axis and including a fluidic measurement chamber (221) adapted to be inserted longitudinally in the housing (120) along the longitudinal insertion axis thereof;
- a receiving surface (211), adapted to be located outside the recess (120) to receive the liquid sample when the main body (220) is inserted in the recess (120), assembled to the main body (220) and comprising a collection orifice (212) in fluidic communication with the measurement chamber (221).

14. Analysis system (1) according to Claim 13, wherein the receiving surface (211) extends in a substantially orthogonal manner to the longitudinal axis of the main body (220).

15. Analysis system (1) according to Claim 14, wherein the recess (120) of the analysis apparatus (100) and the collection device (200) are sized so that when the main body (220) of the collection device is inserted in the recess the distance (D1) between the collection orifice (212) and the measurement chamber (221) is approximately equal to the distance (D2) between the opening of the recess (121) and the measurement chamber (221) and is preferably less than or equal to 2 cm.

16. Analysis system (1) according to Claim 14 or 15, wherein the collection device (200) includes a collection part (210) assembled to the main body (220) and one surface of which forms the receiving surface (211), this collection part (210) being in contact with a wall of the housing (110) at the edge of the opening (121) of the recess when the main body (220) of the collection device is inserted in the recess (120).

17. Analysis system (1) according to any one of Claims 13 to 16, wherein the bearing portion (225) of the main body (220) of the collection device is formed by a shoulder located between a so-called central first part (223) containing a measurement chamber (221) and a distal end of the main body (220) facing the measurement chamber (221).
